# NEW EUROPEAN PATENT SPECIFICATION

(11) **EP 1 504 092 B2**
(45) Date of publication and mention of the opposition decision: **25.06.2014**
(45) Mention of the grant of the patent: 02.11.2011
(21) Application number: 03714379.9
(22) Date of filing: 20.03.2003
(51) Int. Cl.: C12N 9/22, C12N 15/63, C12N 15/90, A01K 67/027, C12N 15/82

(54) **METHODS AND COMPOSITIONS FOR USING ZINC FINGER ENDONUCLEASES TO ENHANCE HOMOLOGOUS RECOMBINATION**
VERFAHREN UND ZUSAMMENSETZUNGEN ZUR VERWENDUNG VON ZINKFINGER-ENDONUKLEASEN ZUR VERBESSERUNG DER HOMOLOGEN REKOMBINATION
PROCEDES ET COMPOSITIONS PERMETTANT D'UTILISER DES ENDONUCLEASES A DOIGTS DE ZINC POUR AMELIORER LA RECOMBINAISON HOMOLOGUE

(30) Priority: 21.03.2002 US 367114 P
(43) Date of publication of application: 09.02.2005
(60) Divisional application: 11165685.6
(73) Proprietor: Sangamo BioSciences, Inc., Richmond, CA 94804 (US)
(72) Inventor: LILJEDAHL, Monika, La Jolla, CA 92037 (US); ASPLAND, Simon, Eric, San Diego, CA 92109 (US); SEGAL, David, J., San Diego, CA 92107 (US)
(74) Representative: Weiss, Wolfgang
(86) International application number: PCT/US2003/009081
(87) International publication number: WO 2003/080809

(56) References cited:
- US-A1- 2004 121 357
- BIBIKOVA M ET AL: "Stimulation of homologous recombination through targeted cleavage by chimeric nucleases" MOLECULAR AND CELLULAR BIOLOGY, AMERICAN SOCIETY FOR MICROBIOLOGY, WASHINGTON, US, vol. 21, no. 1, January 2001 (2001-01), pages 289-297, XP002974229 ISSN: 0270-7306
- BIBIKOVA M ET AL: "Enhancing gene targeting with designed zinc finger nucleases" SCIENCE, AMERICAN ASSOCIATION FOR THE ADVANCEMENT OF SCIENCE,, US, vol. 300, 2 May 2003 (2003-05-02), page 764, XP002974267 ISSN: 0036-8075
- WRIGHT D A ET AL: "High-frequency homologous recombination in plants mediated by zinc-finger nucleases" PLANT JOURNAL, BLACKWELL SCIENTIFIC PUBLICATIONS, OXFORD, GB, vol. 44, no. 4, November 2005 (2005-11), pages 693-705, XP002403198 ISSN: 0960-7412
- SMITH J ET AL: "Requirements for double-strand cleavage by chimeric restriction enzymes with zinc finger DNA-recognition domains" NUCLEIC ACIDS RESEARCH, OXFORD UNIVERSITY PRESS, SURREY, GB, vol. 28, no. 17, 2000, pages 3361-3369, XP002974228 ISSN: 0305-1048
- PUCHTA H ET AL: "HOMOLOGOUS RECOMBINATION IN PLANT CELLS IS ENHANCED BY IN VIVO INDUCTION OF DOUBLE STRAND BREAKS INTO DNA BY A SITE-SPECIFIC ENDONUCLEASE" NUCLEIC ACIDS RESEARCH, OXFORD UNIVERSITY PRESS, SURREY, GB, vol. 21, no. 22, 11 November 1993 (1993-11-11), pages 5034-5040, XP000571587 ISSN: 0305-1048
- SEGAL D J: "The use of zinc finger peptides to study the role of specific factor binding sites in the chromatin environment" METHODS : A COMPANION TO METHODS IN ENZYMOLOGY, ACADEMIC PRESS INC., NEW YORK, NY, US, vol. 26, 2 January 2002 (2002-01-02), pages 76-83, XP002974227 ISSN: 1046-2023
- KIM ET AL: 'Hybrid restriction enzymes: Zinc finger fusions to Fok I cleavage domain' PNAS USA vol. 93, no. 3, 06 February 1996, pages 1156 - 1160, XP002116423
- SEGAL ET AL: 'Endonuclease-induced, targeted homologous extrachromosomal recombination in Xenopus oocytes' PNAS USA vol. 92, no. 3, 31 January 1995, pages 806 - 810, XP002982189

### SEQUENCE LISTING

<110> STELL
   LILJEDAHL, Monika
   ASPLAND, Simon, Eric
   SEGAL, David, J.
<120> METHODS AND COMPOSITIONS FOR USING ZINC FINGER ENDONUCLEASES TO ENHANCE HOMOLOGOUS RECOMBINATION
<130> BIOBANK.010VPC
<150> US 60/367,114
   <151> 2002-03-21
<160> 14
<170> FastSEQ for Windows Version 4.0
<210> 1
   <211> 753
   <212> DNA
<213> Saccharomyces Cerevisiae
<400> 1
   gcaatgtcag acgcttgatg gtaggataat aataattcca aaaaaccatc ataagacatt 60
   cccaatgaca gttgaaggtg agtttgccgc aaaacgcttc atagaagaaa tggagcgctc 120
   taaaggagaa tatttcaact ttgacattga agttagagat ttggattatc ttgatgctca 180
   attgagaatt tctagctgca taagatttgg tccagtactc gcaggaaatg gtgttttatc 240
   taaatttctc actggacgta gtgaccttgt aactcctgct gtaaaaagta tggcttggat 300
   gcttggtctg tggttaggtg acagtacaac aaaagagcca gaaatctcag tagatagctt 360
   ggatcctaag ctaatggaga gtttaagaga aaatgcgaaa atctggggtc tctaccttac 420
   ggtttgtgac gatcacgttc cgctacgtgc caaacatgta aggcttcatt atggagatgg 480
   tccagatgaa aacaggaaga caaggaattt gaggaaaaat aatccattct ggaaagctgt 540
   cacaatttta aagtttaaaa gggatcttga tggagagaag caaatccctg aatttatgta 600
   cggcgagcat atagaagttc gtgaagcatt cttagccggc ttgatcgact cagatgggta 660
   cgttgtgaaa aagggcgaag gccctgaatc ttataaaata gcaattcaaa ctgtttattc 720
   atccattatg gacggaattg tccatatttc aag                               753
<210> 2
   <211> 587
   <212> DNA
   <213> Flavobacterium Okeanokoites
<400> 2
   caactagtca aaagtgaact ggaggagaag aaatctgaac ttcgtcataa attgaaatat 60
   gtgcctcatg aatatattga attaattgaa attgccagaa attccactca ggatagaatt 120
   cttgaaatga aggtaatgga attttttatg aaagtttatg gatatagagg taaacatttg 180
   ggtggatcaa ggaaaccgga cggagcaatt tatactgtcg gatctcctat tgattacggt 240
   gtgatcgtgg atactaaagc ttatagcgga ggttataatc tgccaattgg ccaagcagat 300
   gaaatgcaac gatatgtcga agaaaatcaa acacgaaaca aacatatcaa ccctaatgaa 360
   tggtggaaag tctatccatc ttctgtaacg gaatttaagt ttttatttgt gagtggtcac 420
   tttaaaggaa actacaaagc tcagcttaca cgattaaatc atatcactaa ttgtaatgga 480
   gctgttctta gtgtagaaga gcttttaatt ggtggagaaa tgattaaagc cggcacatta 540
   accttagagg aagtgagacg gaaatttaat aacggcgaga taaactt                 587
<210> 3
   <211> 291
   <212> DNA
   <213> Artificial Sequence
<220>
   <223> SplC framework for producing a zinc finger protein with three fingers (top strand)
<400> 3
   ctcgagcccg gggagaagcc ctatgcttgt ccggaatgtg gtaagtcctt cagtaggaag 60
   gattcgcttg tgaggcacca gcgtacccac acgggtgaaa aaccatataa atgcccagag 120
   tgcggcaaat cttttagtca gtcgggggat cttaggcgtc atcaacgcac tcatactggc 180
   gagaagccat acaaatgtcc ggaatgtggc aagtctttct cggattgtcg tgatcttgcg 240
   aggcaccaac gtactcacac cggtactagt taagtcgacg aggaggagga g           291
<210> 4
   <211> 291
   <212> DNA
   <213> Artificial Sequence
<220>
   <223> SplC framework for producing a zinc finger protein with three fingers (bottom strand)
<400> 4
   ctcctcctcc tcgtcgactt aactagtacc ggtgtgagta cgttggtgcc tcgcaagatc 60
   acgacaatcc gagaaagact tgccacattc cggacatttg tatggcttct cgccagtatg 120
   agtgcgttga tgacgcctaa gatcccccga ctgactaaaa gatttgccgc actctgggca 180
   tttatatggt tttcacccgt tgtgggtacg ctggtgcctc acaagcgaat ccttcctact 240
   gaaggactta ccacattccg gacaagcata gggcttctcc ccgggctcga g           291
<210> 5
   <211> 90
   <212> PRT
   <213> Artificial Sequence
<220>
   <223> SplC framework of zinc finger protein with three fingers
<400> 5
   Leu Glu Pro Gly Glu Lys Pro Tyr Ala Cys Pro Glu Cys Gly Lys Ser
    1               5                  10                  15
   Phe Ser Arg Lys Asp Ser Leu Val Arg His Gln Arg Thr His Thr Gly
               20                  25                  30
   Glu Lys Pro Tyr Lys Cys Pro Glu Cys Gly Lys Ser Phe Ser Gln Ser
           35                  40                  45
   Gly Asp Leu Arg Arg His Gln Arg Thr His Thr Gly Glu Lys Pro Tyr
       50                  55                  60
   Lys Cys Pro Glu Cys Gly Lys Ser Phe Ser Asp Cys Arg Asp Leu Ala
   65                  70                  75                  80
   Arg His Gln Arg Thr His Thr Gly Thr Ser
                   85                  90
<210> 6
   <211> 94
   <212> DNA
   <213> Artificial Sequence
<220>
   <223> PCR Primer
<400> 6
   ctcgagcccg gggagaagcc ctatgcttgt ccggaatgtg gtaagtcctt cagtaggaag 60
   gattcgcttg tgaggcacca gcgtacccac acgg                             94
<210> 7
   <211> 84
   <212> DNA
   <213> Artificial Sequence
<220>
   <223> PCR Primer
<400> 7
   acgcctaaga tcccccgact gactaaaaga tttgccgcac tctgggcatt tatatggttt 60
   ttcacccgtg tgggtacgct ggtg                                        84
<210> 8
   <211> 94
   <212> DNA
   <213> Artificial Sequence
<220>
   <223> PCR Primer
<400> 8
   cttttagtca gtcgggggat cttaggcgtc atcaacgcac tcatactggc gagaagccat 60
   acaaatgtcc ggaatgtggc aagtctttct cgga                            94
<210> 9
   <211> 91
   <212> DNA
   <213> Artificial Sequence
<220>
   <223> PCR Primer
<400> 9
   ctcctcctcc tcgtcgactt aactagtacc ggtgtgagta cgttggtgcc tcgcaagatc 60
   acgacaatcc gagaaagact tgccacattc c 91
<210> 10
   <211> 27
   <212> DNA
   <213> Artificial Sequence
<220>
   <223> Target sequence for HO endonuclease domain
<221> misc_feature
   <222> 2, 3, 5, 6, 8, 9, 11, 12, 13, 14, 15, 16, 17, 18, 19, 20
   <223> n = A,T,C or G
<400> 10
   rnnrnnrnnr nnnnnnnnnn ynnynny 27
<210> 11
   <211> 18
   <212> DNA
   <213> Artificial Sequence
<220>
   <223> Target sequence for Fok I containing ZFEs
<221> misc_feature
   <222> 2, 3, 5, 6, 8, 9, 11, 12, 14, 15, 17, 18
   <223> n = A,T,C or G
<400> 11
   rnnrnnrnnr nnrnnrnn 18
<210> 12
   <211> 9
   <212> DNA
   <213> Artificial Sequence
<220>
   <223> Zinc finger recognition sequence
<400> 12
   gtggcagcc 9
<210> 13
   <211> 5
   <212> PRT
   <213> Artificial Sequence
<220>
   <223> Zinc Finger protein consensus linker sequence
<400> 13
   Thr Gly Glu Lys Pro
   1 5
<210> 14
   <211> 16
   <212> DNA
   <213> Artificial Sequence
<220>
   <223> Stopper sequence that introduces stop codon in 3 reading frames of target sequence
<400> 14
   actagttaac tgatca 16

## Description

### Background of the Invention

For scientists studying gene function, the introduction of genetic modifications in the germ-line of live animals was both a major breakthrough in biology, and also an invaluable tool (Jaenisch, Science 2405, 1468-74 (1988)). The mouse has been the favorite model of scientists studying mammals. The mouse has also been the only species for which large scale analysis has been possible. Using mice it is not only possible to add genes, but also to delete ("knock-out"), replace, or modify genes (Capecchi, "Altering the genome by homologous, recombination," Science 244, 1288-1292 (1989)). Two key technologies facilitated the generation of genetically modified mice:

First, methods were developed which allowed embryonic stem cells (ES), which can colonize all the tissues of a host embryo, including its germ line, to be grown in culture. (Evans, "Establishment in culture of pluripotential cells from mouse embryos," Nature 292(5819):154-6 (Jul. 9, 1981)).

Second, methods for utilizing homologous recombination between an incoming DNA and its cognate chromosomal sequence ("targeting") to introduce a desired nucleic acid into ES cells to generate genetically modified mice were developed (Kuehn et al., "A potential animal model for Lesch-Nyhan syndrome through introduction of HPRT mutations into mice," Nature 25:326(6110):295-8 (Mar. 19, 1987)).

By using these techniques, genetically modified mice, including mice carrying null mutations in any desired gene have become a reality. For some genes this is the ultimate way to find gene function.

Initially, these techniques were used to simply knock genes out, but in recent years, as a result of further refinement, their application has become broader. Examples of the other types of genetic modifications that can be created include subtle mutations (point mutations, micro deletions or insertions, etc.) and more dramatic mutations, such as large deletions, duplications and translocations. Also, it has also become possible to create conditional mutations in which a gene is initially present, but is removed at a later point in development. This has facilitated the study of the later role of genes which are critical for normal embryonic development (Baubonis et al., "Genomic targeting with purified Cre recombinase," Nucleic Acids Res. 21(9):2025-9 (May 11, 1993); Gu et al., "Independent Control of Immunoglobulin Switch Recombination at Individual Switch Regions Evidenced Through Cre-IoxP-mediated Gene Targeting," Cell 73:1155 (1993)).

However, the generation of transgenic mice or genetically modified mice using ES cells is still relatively inefficient, technically demanding, and costly. The ability to generate genetically modified mice using ES technology is a result of the fact that ES cells can be maintained in culture virtually indefinitely remaining totipotent. Because ES cells can be maintained in culture for long periods of time, it is possible to obtain a sufficient number of ES cells in which a desired homologous recombination event has occurred despite the fact that homologous recombination is a very inefficient process.

Because embryonic stem cell lines are not yet available for mammals other than the mouse, the generation of genetically modified mammals other than mice has to be carried out using somatic cells such as fetal fibroblasts, skin fibroblasts or mammary gland cells (Ridout III et al., "Nuclear cloning and epigenetic reprogramming of the genome," Science 293(5532):1093-8 (Aug. 10, 2001)). In such techniques, a genetically modified somatic cell is generated and the nucleus from the genetically modified cell then is transferred (nuclear transfer) into a fertilized oocyte.

In contrast to ES cells, the somatic cells, which provide the nuclei used in nuclear transfer, only divide in culture for a limited time. This consequently makes homologous recombination in animals without ES cells a very challenging undertaking, although not impossible, as discussed below.

The technology to engineer genetic manipulations in other animals is just starting to develop. Dolly the sheep was the very first example of any animal cloned by nuclear transfer from a differentiated, adult, somatic cell. (Campbell et al., "Sheep cloned by nuclear transfer from a cultured cell line," Nat. 380, 64-66 (1996)). Dolly was an identical copy of another sheep with no genetic alterations to her genome, such as additions or deletions of any genes. This signal accomplishment was achieved 6 years ago. Since then, mice, cattle, goats, pigs and a cat all have been cloned by nuclear transfer (Shin et al., "Cell biology: A cat cloned by nuclear transplantation," Nature 415 (6874):859 (2002)).

In another example, Human Factor IX genes were randomly inserted into fetal sheep somatic cell nuclei and over-expressed. The engineered nuclei were subsequently used to clone sheep (Schnieke et al., "Human factor IX transgenic sheep produced by transfer of nuclei from transfected fetal fibroblasts," Sci. 278, 2130-2133 (1997)). Transgenic animals with site-specific gene inserts have recently been achieved in sheep, with the targeted insertion at the sheep α1 (alpha-1) procollagen locus (McCreath et al. "Production of gene-targeted sheep by nuclear transfer from cultured somatic cells," Nature 405, 1066-1069 (2000)).

Further, progress has been made in the production of viable cloned swine from genetically engineered somatic cell nuclei. One of the two alleles coding for the α (alpha) Galactosyl transferase gene has been deleted from somatic swine cell nuclei, and the nuclei from these cells were transferred to oocytes to produce viable piglets. (Lai et al., "Production of {alpha}-1,3-Galactosyltransferase Knockout Pigs by Nuclear Transfer Cloning," Science (2002)) and (Liangxue et al., "Production of α-1,3-Galactosyltransferase Knockout Pigs by Nuclear Transfer Cloning," Science 10.1126 (published online January 3, 2002); "Second Group Announces 'Knock Out' Cloned Pigs," Scientific American (PPL, Jan 4, 2002)). The production of apparently normal clones from somatic cell nuclei indicates that this approach is feasible for the creation of genetically engineered animals.

The generation of animals by nuclear transfer of somatic cell nuclei is very inefficient. Hundreds or thousands of transfers are required in order to produce a few viable offspring. Somatic cell nuclear transfer also leads to physiological problems in many of the viable offspring with the offspring suffering from multiple types of organ failure including unusually large organs, heart defects, etc. Although some clones are apparently normal, others exhibit one or more of the symptoms of this syndrome. It is thought that the chromosomal modification patterns ("imprinting") (Ferguson-Smith, "Imprinting and the epigenetic asymmetry between parental genomes," Science 10;293(5532):1086-9 (Aug. 2001)) that naturally occurs in germ cells, following fertilization may not occur efficiently during the somatic nuclear cloning procedures. The lack of proper imprinting is likely to cause the syndromes observed in many of the clones that survive to birth.

Breaking DNA using site specific endonucleases can increase the rate of homologous recombination in the region of the breakage. This has been demonstrated a number of times with the I-Sce I endonuclease from the yeast *Saccharomyces cerevisiae.* I-Sce I is an endonuclease encoded by a mitochondrial intron which has an 18 bp recognition sequence, and therefore a very low frequency of recognition sites within a given DNA, even within large genomes (Thierry et al., "Cleavage of yeast and bacteriophage T7 genomes at a single site using the rare cutter endonuclease I-Sce I," Nucleic Acids Res. 19 (1):189-190 (1991)). The infrequency of cleavage sites recognized by I-SceI makes it suitable to use for enhancing homologous recombination.

The recognition site for I-Sce I has been introduced into a range of different systems. Subsequent cutting of this site with I-Sce I increases homologous recombination at the position where the site has been introduced. Enhanced frequencies of homologous recombination have been obtained with I-Sce I sites introduced into the extra-chromosomal DNA in *Xenopus* oocytes, the mouse genome, and the genomic DNA of the tobacco plant *Nicotiana plumbaginifolia.* See, for example, Segal et al., "Endonuclease-induced, targeted homologous extrachromosomal recombination in Xenopus oocytes," Proc.Nati.Acad.Sci.U.S.A. 92 (3):806-810 (1995); Choulika et al., "Induction of homologous recombination in mammalian chromosomes by using the I-SceI system of Saccharomyces cerevisiae," Mol.Cell Biol. 15 (4):1968-1973 (1995); and Puchta et al., "homologous recombination in plant cells is enhanced by in vivo induction of double strand breaks into DNA by a site-specific endonuclease," Nucleic Acids Res. 21 (22):5034-5040 (1993).

The limitation of the I-Sce I approach is that the I-Sce I recognition site has to be introduced by standard methods of homologous recombination at the desired location prior to the use of I-Sce-I endonuclease to enhance homologous recombination at that site.

Thus, there is a need for more efficient methods for generating genetically modified organisms and, in particular, genetically modified organisms in species where ES cells are not available. More efficient methods of generating genetically modified organisms would be advantageous for scientists studying basic and applied biology. Moreover, methods that permit efficient genetic modification, including removal of genes in larger animals, would be extremely useful in agriculture, biotechnology and human healthcare.

### Summary of the Invention

The present invention is defined by the appended set of claims.

Some embodiments of the present invention are described below. However, it will be appreciated that the scope of the present invention is defined solely by the appended claims. Accordingly, other embodiments which will be apparent to those of skill ordinary in the art in view of the disclosure herein are also within the scope of this invention.

Described are methods of generating a genetically modified cell. The methods can include providing a primary cell containing an endogenous chromosomal target DNA sequence in which it is desired to have homologous recombination occur. The methods also can include providing a zinc finger endonuclease (ZFE) that includes an endonuclease domain that cuts DNA, and a zinc finger domain that includes a plurality of zinc fingers that bind to a specific nucleotide sequence within the endogenous chromosomal target DNA in the primary cell. Further, the methods can include contacting the endogenous chromosomal target DNA sequence with the zinc finger endonuclease in the primary cell such that the zinc finger endonuclease cuts both strands of a nucleotide sequence within the endogenous chromosomal target DNA sequence in the primary cell, thereby enhancing the frequency of homologous recombination in the endogenous chromosomal target DNA sequence. The methods also include providing a nucleic acid comprising a sequence homologous to at least a portion of said endogenous chromosomal target DNA such that homologous recombination occurs between the endogenous chromosomal target DNA sequence and the nucleic acid. The zinc finger endonuclease further can include a protein tag to purify the resultant protein. For example, the protein tag can be HA tag, FLAG-tag, GST-tag, c-myc, His-tag, and the like. The contacting step can include transfecting the primary cell with a vector that includes a cDNA encoding the zinc finger endonuclease, and expressing a zinc finger endonuclease protein in the primary cell. In other embodiments the contacting step can include injecting a zinc finger endonuclease protein into said primary cell, for example by microinjection. The endonuclease domain is an HO endonuclease. The zinc finger domain that binds to a specific nucleotide sequence within the endogenous chromosomal target DNA includes according to the invention, five or more zinc fingers. It is described that the zinc finger domain that binds to a specific nucleotide sequence within the endogenous chromosomal target DNA can include three or more zinc fingers. Each of the plurality of zinc fingers can bind, for example, to the sequence G/ANN. The cell can be from a plant, a mammal, a marsupial, teleost fish, an avian, and the like. In preferred embodiments, the mammal can be a human, a non-human primate, a sheep, a goat, a cow, a rat a pig, and the like. In other preferred embodiments, the mammal can be a mouse. In other preferred embodiments, the teleost fish can be a zebrafish. In other preferred embodiments the avian can be a chicken, a turkey and the like. In more preferred embodiments, the primary cell can be from an organism in which totipotent stem cells are not available.

Bibikova et al (Molecular and Cellular Biology, Jan. 2001, p. 289-297) discloses homologous recombination through targeted cleavage by chimeric nucleases.

Bibikova et al (Science, Vol. 300, 2003, p. 764) relates to enhanced gene targeting with designed zinc finger nucleases in Drosophila.

Puchta et al (Nucleic Acid Research, 1993, Vol. 21, p. 5034-5040) relates to homologous recombination in plant cells by induction of double-strand breaks into DNA by a site-specific endonuclease.

WO 03/087341 describes targeted chromosomal mutagenesis using zinc finger nucleases.

Also described are methods of designing a sequence specific zinc finger endonuclease capable of cleaving DNA at a specific location. The methods include identifying a first unique endogenous chromosomal nucleotide sequence adjacent to a second nucleotide sequence at which it is desired to introduce a double-stranded cut; and designing a combination of sequence specific zinc finger endonucleases that are capable of cleaving DNA at a specific location, the zinc finger endonucleases including a plurality of zinc fingers which bind to the unique endogenous chromosomal nucleotide sequence and an endonuclease which generates a double-stranded cut at the second nucleotide sequence. Described is further that the designing step can include designing a zinc finger endonuclease that includes a plurality of zinc fingers that are specific for said endogenous nucleic acid sequence and an endonuclease which generates a double-stranded cut at said second nucleotide sequence.

Still further embodiments of the invention relate to zinc finger endonuclease for cutting a specific DNA sequence to enhance the rate of homologous recombination. The zinc finger endonucleases include an endonuclease domain and a zinc finger domain specific for an endogenous chromosomal DNA sequence. In other embodiments, the zinc finger endonucleases also can include a purification tag. The endonuclease domain is HO endonuclease. The zinc finger domain specific for said endogenous chromosomal DNA sequence includes at least five zinc fingers, and more preferably six zinc fingers. The purification tag can include HA tag, FLAG-tag, GST-tag, c-myc, His-tag, and the like.

Additional embodiments of the invention relate to methods of generating a genetically modified animal in which a desired nucleic acid has been introduced. The methods include obtaining a primary cell that includes an endogenous chromosomal target DNA sequence into which it is desired to introduce said nucleic acid; generating a double-stranded cut within said endogenous chromosomal target DNA sequence with a zinc finger endonuclease comprising a zinc finger domain that binds to an endogenous target nucleotide sequence within said target sequence and an endonuclease domain; introducing an exogenous nucleic acid that includes a sequence homologous to at least a portion of the endogenous chromosomal target DNA into the primary cell under conditions which permit homologous recombination to occur between the exogenous nucleic acid and the endogenous chromosomal target DNA; and generating an animal from the primary cell in which homologous recombination has occurred. The zinc finger domain includes at least 5 zinc fingers. The animal can be, for example, a mammal, a marsupial, teleost fish, an avian, and the like. In preferred embodiments, the mammal can be, for example, a human, a non-human primate, a sheep, a goat, a cow, a rat a pig, and the like. In other embodiments the mammal can be a mouse. The teleost fish can be a zebrafish in some embodiments. In other embodiments the avian can be a chicken, a turkey, and the like. The homologous nucleic acid can include a nucleotide sequence can be a nucleotide sequence which disrupts a gene after homologous recombination, a nucleotide sequence which replaces a gene after homologous recombination, a nucleotide sequence which introduces a point mutation into a gene after homologous recombination, a nucleotide sequence which introduces a regulatory site after homologous recombination, and the like. In preferred embodiments the regulatory site can include a LoxP site.

Also described are genetically modified animals made according to the described methods.

Further embodiments relate to methods of generating a genetically modified plant in which a desired nucleic acid has been introduced. The methods can include obtaining a plant cell that includes an endogenous target DNA sequence into which it is desired to introduce the nucleic acid; generating a double-stranded cut within the endogenous target DNA sequence with a zinc finger endonuclease that includes a zinc finger domain that binds to an endogenous target nucleotide sequence within the target sequence and an endonuclease domain; introducing an exogenous nucleic acid that includes a sequence homologous to at least a portion of the endogenous target DNA into the plant cell under conditions which permit homologous recombination to occur between the exogenous nucleic acid and the endogenous target DNA; and generating a plant from the plant cell in which homologous recombination has occurred. Also disclosed are genetically modified cells and plants made according to the method described above and herein.

### Brief Description of the Drawings

Figure 1 illustrates the sequence of the Pst I-Bgl II fragment of the HO endonuclease (SEQ ID NO: 1).

Figure 2 illustrates a sequence for the Fok I endonuclease domain used in chimeric endonucleases (SEQ ID NO: 2) (for comparison)

Figure 3 illustrates exemplary zinc finger endonuclease strategies.

Figure 4 illustrates a Sp1C framework for producing a zinc finger protein with three fingers (SEQ ID NOs: 3-5).

Figure 5 illustrates exemplary primers used to create a zinc finger domain with three fingers (SEQ ID NOs: 6-9).

Figure 6 illustrates a method for comparison.

Figure 7 illustrates a "Positive/Negative" homologous recombination construct.

Figure 8 illustrates a "Gene Trap" homologous recombination construct.

Figure 9 illustrates an "Over-lapping" homologous recombination construct.

### Detailed Description of the Preferred Embodiment

The present invention provides more efficient methods for generating genetically modified cells which can be used to obtain genetically modified organisms. In some embodiments of the present invention, a cell capable of generating a desired organism is obtained. Preferably the cell is a primary cell. The cell contains an endogenous nucleotide sequence at or near which it is desired to have homologous recombination occur in order to generate an organism containing a desired genetic modification. The frequency of homologous recombination at or near the endogenous nucleotide sequence is enhanced by cleaving the endogenous nucleotide sequence in the cell with an zinc finger endonuclease. Both strands of the endogenous nucleotide sequence are cleaved by the zinc finger endonuclease. A nucleic acid comprising a nucleotide sequence homologous to at least a portion of the chromosomal region containing or adjacent to the endogenous nucleotide sequence at which the zinc finger endonuclease cleaves is introduced into the cell such that homologous recombination occurs between the nucleic acid and the chromosomal target sequence. Thereafter, a cell in which the desired homologous recombination event has occurred may be identified and used to generate a genetically modified organism using techniques such as nuclear transfer.

Zinc finger endonucleases (ZFEs) are used to enhance the rate of homologous recombination in cells. Preferably, the cells are from species in which totipotent stem cells are not available, but in other embodiments the cells may be from an organism in which totipotent stem cells are available, and, in some embodiments, the cell may be a totipotent stem cell. Preferably, the cell is a primary cell, but in some embodiments, the cell may be a cell from a cell line. For example, in some embodiments, the cells may be from an organism such as a mammal, a marsupial, a teleost fish, an avian and the like. The mammal may be a human, a non-human primate, a sheep, a goat, a cow, a rat, a pig, and the like. In other embodiments, the mammal can be a mouse. In some embodiments, the teleost fish may be a zebrafish. In other embodiments the avian may be a chicken, a turkey, and the like.

The cells may be any type of cell which is capable of being used to generate a genetically modified organism or tissue. For example, in some embodiments, the cell may be primary skin fibroblasts, granulosa cells, primary fetal fibroblasts, stem cells, germ cells, fibroblasts or non-transformed cells from any desired organ or tissue. In some embodiments, the cell may be a cell from which a plant may be generated, such as for example, a protoplast.

In some embodiments of the present invention, a ZFE is used to cleave an endogenous chromosomal nucleotide sequence at or near which it is desired to introduce a nucleic acid by homologous recombination. The ZFE comprises a zinc finger domain which binds near the endogenous nucleotide sequence at which is to be cleaved and an endonuclease domain which cleaves the endogenous chromosomal nucleotide sequence. As mentioned, above, cleavage of the endogenous chromosomal nucleotide sequence increases the frequency of homologous recombination at or near that nucleotide sequence. In some embodiments, the ZFEs can also include a purification tag which facilitates the purification of the ZFE.

The endonuclease used according to the invention is HO endonuclease. The endonuclease domain is fused to the heterologous DNA binding domain (such as a zinc finger DNA binding domain) such that the endonuclease will cleave the endogenous chromosomal DNA at the desired nucleotide sequence. It is described for comparison that the endonuclease domain may be from the Fok I endonuclease.

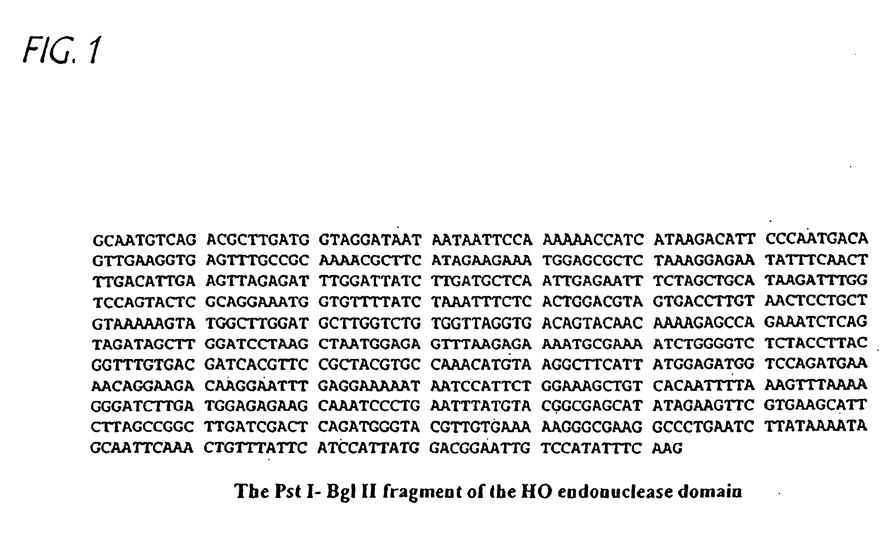

The HO endonuclease domain from *Saccharomyces cerevisiae* is encoded by a 753 bp Pst I-Bgl II fragment of the HO endonuclease cDNA available on Pubmed (Acc # X90957). The HO endonuclease cuts both strands of DNA (Nahon et al., "Targeting a truncated Ho-endonuclease of yeast to novel DNA sites with foreign zinc fingers," Nucleic Acids Res. 26 (5):1233-1239 (1998)). Figure 1 illustrates the sequence of the Pst I-Bgl II fragment of the HO endonuclease cDNA (SEQ ID NO: 1) which may be used in the ZFEs of the present invention. *Saccharomyces cerevisiae* genes rarely contain any introns, so, if desired, the HO gene can be cloned directly from genomic DNA prepared by standard methods. For example, if desired, the HO endonuclease domain can be cloned using standard PCR methods.

For comparison the Fok I (Flavobacterium okeanokoites) endonuclease may be fused to a heterologous DNA binding domain. The Fok I endonuclease domain functions independently of the DNA binding domain and cuts a double stranded DNA only as a dimer (the monomer does not cut DNA) (Li et al., "Functional domains in Fok I restriction endonuclease," Proc.Noti.Acad.Sci.U.S.A 89 (10):4275-4279 (1992), and Kim et al., "Hybrid restriction enzymes: zinc finger fusions to Fok I cleavage domain," Proc.Natl.Acad.Sci. U.S.A 93 (3):1156-1160 (1996)). Therefore, in order to create double stranded DNA breaks, two ZFEs are positioned so that the Fok I domains they contain dimerise.

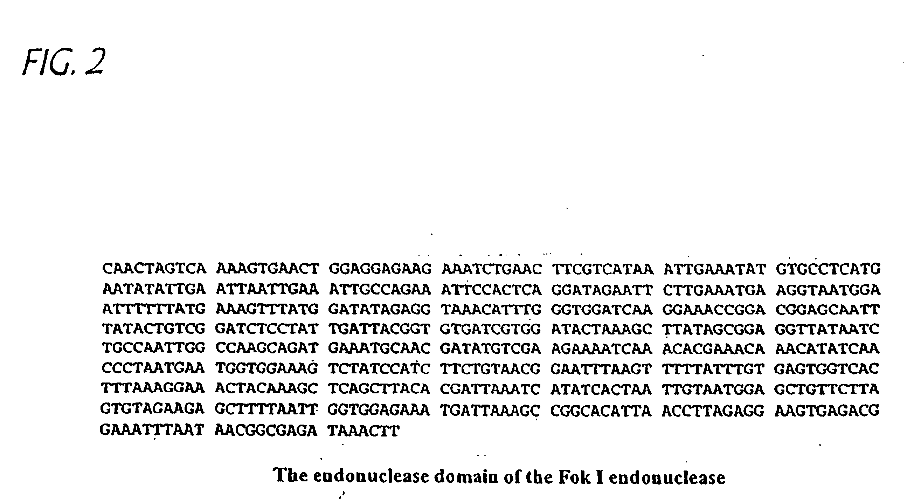

The Fok I endonuclease domain can be cloned by PCR from the genomic DNA of the marine bacteria *Flavobacterium okeanokoites* (ATCC) prepared by standard methods. The sequence of the Fok I endonuclease is available on Pubmed (Acc # M28828 and Acc # J04623). Figure 2 depicts the sequence of the Fok I endonuclease domain (SEQ ID NO: 2).

As mentioned above, the ZFE includes a zinc finger domain with specific binding affinity for a desired specific target sequence. The ZFE specifically binds to an endogenous chromosomal DNA sequence. The specific nucleic acid sequence or more preferably specific endogenous chromosomal sequence can be any sequence in a nucleic acid region where it is desired to enhance homologous recombination. For example, the nucleic acid region may be a region which contains a gene in which it is desired to introduce a mutation, such as a point mutation or deletion, or a region into which it is desired to introduce a gene conferring a desired phenotype.

There are a large number of naturally occurring zinc finger DNA binding proteins which contain zinc finger domains that may be incorporated into a ZFE designed to bind to a specific endogenous chromosomal sequence. Each individual "zinc finger" in the ZFE recognizes a stretch of three consecutive nucleic acid base pairs. The ZFE may have a variable number of zinc fingers. For example, ZFEs with between one and six zinc fingers can be designed. In other examples, more than six fingers can be used. ZFEs according to the invention comprise five or more zinc fingers. A two finger protein has a recognition sequence of six base pairs, a three finger protein has a recognition sequence of nine base pairs and so on. Therefore, the ZFEs used in the methods of the present invention may be designed to recognize any desired endogenous chromosomal target sequence, thereby avoiding the necessity of introducing a cleavage site recognized by the endonuclease into the genome through genetic engineering

The ZFE protein is designed and/or constructed to recognize a site which is present only once in the genome of a cell. One ZFE protein is designed and made with at least five zinc fingers. Also, more than one ZFE protein can be designed and made so that collectively the ZFEs have five zinc fingers (i.e. a ZFE having two zinc fingers may complex with a ZFE having 3 zinc fingers to yield a complex with five zinc fingers). Five is used here only as an exemplary number. Any other number of fingers can be used. Five zinc fingers, either individually or in combination, have a recognition sequence of at least fifteen base pairs. Statistically, a ZFE with 5 fingers will cut the genome once every 415 (about 1 x 10⁹) base pairs, which should be less than once per average size genome. In more preferred embodiments, an individual protein or a combination of proteins with six zinc fingers can be used. Such proteins have a recognition sequence of 18 bp.

Appropriate ZFE domains can be designed based upon many different considerations. For example, use of a particular endonuclease may contribute to design considerations for a particular ZFE. As an exemplary illustration, the yeast HO domain can be linked to a single protein that contains six zinc fingers because the HO domain cuts both strands of DNA. Further discussion of the design of sequence specific ZFEs is presented below.

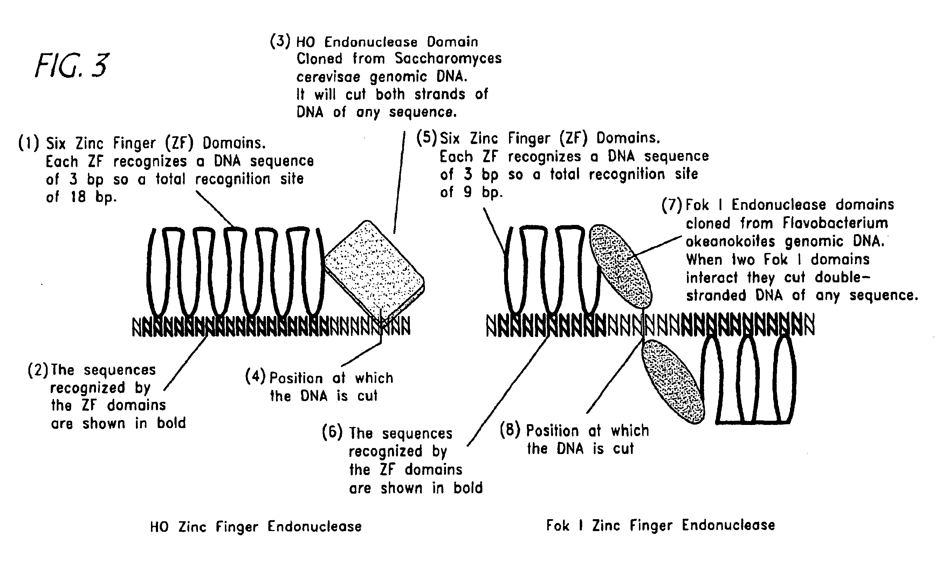

It is described for comparison that the Fok I endonuclease domain only cuts double stranded DNA as a dimer. Therefore, two ZFE proteins can be made and used in the method described. These ZFEs can each have a Fok I endonuclease domain and a zinc finger domain with three fingers. They can be designed so that both Fok I ZFEs bind to the DNA and dimerise. In such cases, these two ZFEs in combination have a recognition site of 18 bp and cut both strands of DNA. Figure 3 illustrates examples of a ZFE that includes an HO endonuclease (inventive), and ZFEs using the Fok I endonuclease (for comparison). Each ZFE in Figure 3 has an 18 bp recognition site and cuts both strands of double stranded DNA.

For example, Figure 3 illustrates a ZFE that includes an HO endonuclease. Figure 3 includes (1) six zinc finger (ZF) domains, each of which recognizes a DNA sequence of 3 bp resulting in a total recognition site of 18 bp. (2) The sequence recognized by the ZF domains is shown by bolded "N"s. (3) The ZFs are attached to an HO Endonuclease domain cloned from *Saccharomyces cerevisiae* genomic DNA. The HO endonuclease domain cuts both strands of DNA of any sequence, and the position of the cut is shown (4).

Figure 3 also depicts a ZFE that includes a Fok I zinc finger endonuclease. The ZFE includes (5) a dimer with six zinc finger (ZF) domains, each of which recognizes a DNA sequence of 3 bp, resulting in a total recognition sit of 9 bp. (6) The sequences recognized by the ZF domains are shown by bolded "N"s. (7) The ZFs are each attached to a Fok I endonuclease domain cloned from *Flavobacterium okeanokoites* genomic DNA. When two Fok I domains interact they cut double-stranded DNA of any sequence. The Fok 1 endonuclease domains cut at the shown position (8).

The particular zinc fingers used in the ZFE will depend on the target sequence of interest. A target sequence in which it is desired to increase the frequency of homologous recombination can be scanned to identify binding sites therein which will be recognized by the zinc finger domain of a ZFE. The scanning can be accomplished either manually (for example, by eye) or using DNA analysis software, such as MacVector (Macintosh) or Omiga 2.0 (PC), both produced by the Genetics Computer Group. For comparison or a pair of Fok I containing ZFEs, two zinc finger proteins, each with three fingers, bind DNA in a mirror image orientation, with a space of 6 bp in between the two. For example, the sequence that is scanned for can be 5'-G/A N N G/A N N G/A N N N N N N N N N N C/T N N C/T N N C/T-3' (SEQ ID NO: 10). If according to the invention a six finger protein with an HO endonuclease domain attached is used, then the desired target sequence can be 5'- G/A N N G/A N N G/A N N G/A N N G/A N N G/A N N-3' (SEQ ID NO: 11), for example. In these examples, if "N" is any base pair, then all of the zinc fingers that bind to any sequence "GNN" and "ANN" are already determined (Segal et al., "Toward controlling gene expression at will: selection and design of zinc finger domains recognizing each of the 5'-GNN-3' DNA target sequences," Proc.Natl.Acad.Sci.U.S.A 96 (6):2758-2763 (1999), and Dreier et al., "Development of zinc finger domains for recognition of the 5'-ANN-3' family of DNA sequences and their use in the construction of artificial transcription factors," J.Biol.Chem. 276 (31):29466-29478 (2001)).

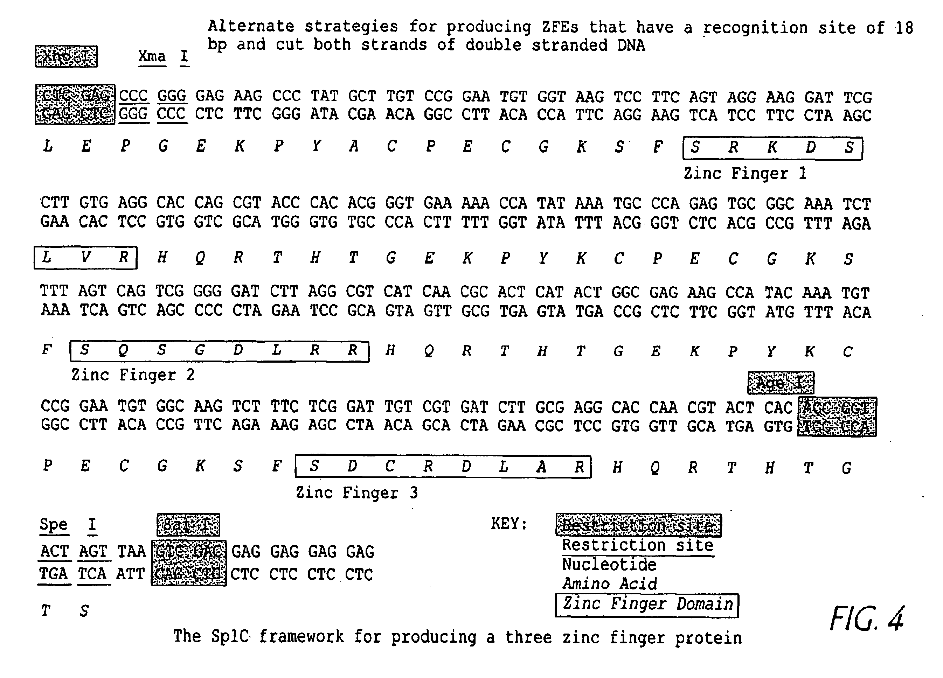

The sequence encoding the identified zinc fingers can be cloned into a vector according well known methods in the art. In one example, Figure 4 illustrates one possible peptide framework into which any three zinc fingers that recognize consecutive base pair triplets can be cloned. Any individual zinc finger coding region can be substituted at the positions marked for zinc finger 1, zinc finger 2 and zinc finger 3. In this particular example zinc finger 1 recognizes "GTG", zinc finger 2 "GCA" and zinc finger 3 "GCC", so all together this protein will recognize "GTGGCAGCC" (SEQ ID NO: 12). Restriction sites are present on either side of this sequence to facilitate cloning. The backbone peptide in this case is that of SpIC, a consensus sequence framework based on the human transcription factor Spl (Desjarlais et al., "Use of a zinc-finger consensus sequence framework and specificity rules to design specific DNA binding proteins," Proc.Natl.Acad.Sci.U.S.A 90 (6):2256-2260 (1993)).

For comparison, SpIC is a three finger network and as such can be the zinc finger DNA binding domain that is linked to the Fok I endonuclease domain. Using the restriction sites Age I and Xma I two three-finger coding regions can be joined to form a six-finger protein with the same consensus linker (TGEKP; SEQ ID NO: 13) between all fingers. This technique is described in (Desjarlais et al., "Use of a zinc-finger consensus sequence framework and specificity rules to design specific DNA binding proteins," Proc.Natl.Acad.Sci.U.S.A 90 (6):2256-2260 (1993).) This six finger framework can be the zinc finger DNA binding domain that is linked to a desired endonuclease domain. The skilled artisan will appreciate that many other frameworks can be used to clone sequences encoding a plurality of zinc fingers.

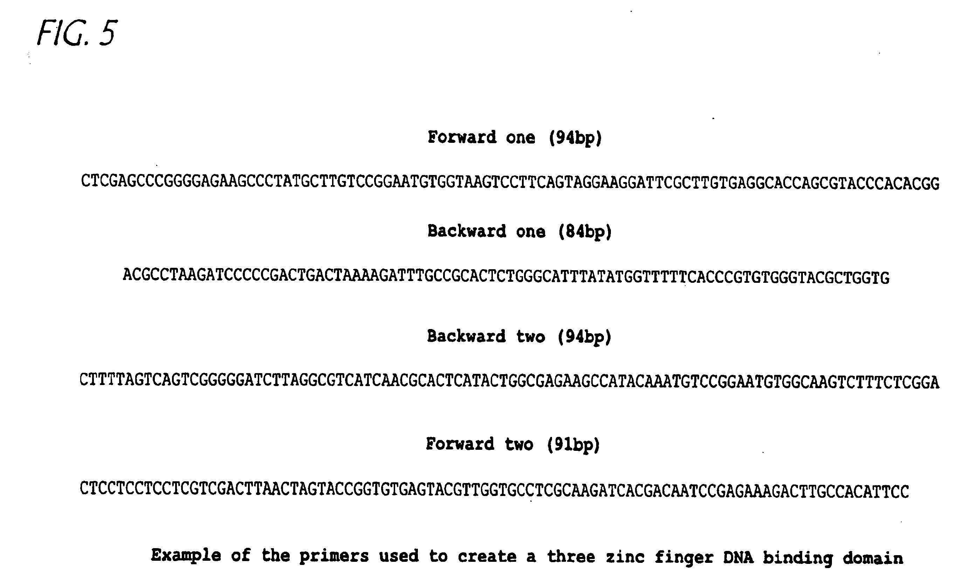

The sequence in Figure 4 can be constructed using standard PCR methods. Figure 5 illustrates exemplary PCR primers that can be used. Two 94 bp "forward" primers (SEQ ID NOs: 6 and 8) can encode the 5' strand, and two "backward" primers that overlap these "forward" primers, one 84 bp (SEQ ID NO: 7) the other 91 bp (SEQ ID NO: 9), can encode the 3' strand. These primers can provide both the primers and the template when mixed together in a PCR reaction.

It will be appreciated that the zinc fingers in the ZFEs used in the methods of the present invention may be any combination of zinc fingers which recognize the desired binding site. The zinc fingers may come from the same protein or from any combination of heterologous proteins which yields the desired binding site.

A nucleotide sequence encoding a ZFE with the desired number of fingers fused to the desired endonuclease is cloned into a desired expression vector. There are a number of commercially available expression vectors into which the nucleotide sequence encoding the ZFE can be cloned. The expression vector is then introduced into a cell capable of producing an active ZFE. For example, the expression vector may be introduced into a bacterial cell, a yeast cell, an insect cell or a mammalian cell. Preferably, the cell lacks the binding site recognized by the ZFE. Alternatively, the cell may contain the binding site recognized by the ZFE but the site may be protected from cleavage by the endonuclease through the action of cellular enzymes.

In other embodiments, the ZFE can be expressed or produced in a cell free system such as TNT Reticulocyte Lysate. The produced ZFE can be purified by any appropriate method, including those discussed more fully herein. In preferred embodiments, the ZFE also includes a purification tag which facilitates purification of the ZFE. For example, the purification tag may be the maltose binding protein, myc epitope, a poly-histidine tag, HA tag, FLAG-tag, GST-tag, or other tags familiar to those skilled in the art. In other embodiments, the purification tag may be a peptide which is recognized by an antibody which may be linked to a solid support such as a chromatography column.

Many commercially available expression systems include purification tags, which can be used with the embodiments of the invention. Three examples of this are pET-14b (Novagen) which produces a Histidine tagged protein produced under the control of T7 polymerase. This vector is suitable for use with TNT Reticulocyte Lysate (Promega). The pMal system (New England Biolabs) which produces maltose binding protein tagged proteins under the control of the malE promoter in bacteria may also be used. The pcDNA vectors (Invitrogen) which produce proteins tagged with many different purification tags in a way that is suitable for expression in mammalian cells may also be used.

The ZFE produced as described above is purified using conventional techniques such as a chromatography column containing moieties thereon which bind to the purification tag. The purified ZFE is then quantified and the desired amount of ZFE is introduced into the cells in which it is desired to enhance the frequency of homologous recombination. The ZFE may be introduced into the cells using any desired technique. In a preferred embodiment, the ZFE is microinjected into the cells.

Alternatively, rather than purifying the ZFE and introducing it into the cells in which it is desired to enhance the frequency of homologous recombination, the ZFE may be expressed directly in the cells. In such embodiments, an expression vector containing a nucleotide sequence encoding the ZFE operably linked to a promoter is introduced into the cells. The promoter may be a constitutive promoter or a regulated promoter. The expression vector may be a transient expression vector or a vector which integrates into the genome of the cells.

A recombination vector comprising a 5' region homologous to at least a portion of the chromosomal region in which homologous recombination is desired and a 3' region homologous to at least a portion of the chromosomal region in which homologous recombination is introduced into the cell. The lengths of the 5' region and the 3' region may be any lengths which permit homologous recombination to occur. The recombination also contains an insertion sequence located between the 5' region and the 3' region. The insertion sequence is a sequence which is desired to be introduced into the genome of the cell.

For example, in some embodiments, the insertion sequence may comprise a gene which is desired to be introduced into the genome of the cell. In some embodiments, the gene may be operably linked to a promoter in the recombination vector. Alternatively, in other embodiments, the gene may become operably linked to a promoter in the adjacent chromosomal region after homologous recombination has occurred. In some embodiments the gene may be a gene from the same organism as the cells in which it is to be introduced. For example, the gene may be a wild type gene which rescues a genetic defect in the cell after it is introduced through homologous recombination. Alternatively, the gene may confer a desired phenotype, such as disease resistance or enhanced nutritional value, on the organism in which it is introduced.

In other embodiments, the gene may be from a different organism than the cell into which it is to be introduced. For example, the gene may encode a therapeutically beneficial protein from an organism other than the organism from which the cell was obtained. In some embodiments, for example, the gene may encode a therapeutically beneficial human protein such as a growth factor, hormone, or tumor suppressor.

In some embodiments, the insertion sequence introduces a point mutation into an endogenous chromosomal gene after homologous recombination has occurred. The point mutation may disrupt the endogenous chromosomal gene or, alternatively, the point mutation may enhance or restore its activity.

In other embodiments, the insertion sequence introduces a deletion into an endogenous chromosomal gene after homologous recombination has occurred. In such embodiments, the insertion sequence may "knock out" the target gene.

In some embodiments, it may be desired to replace, disrupt, or knock-out both chromosomal copies of the target gene or to introduce two copies of a desired nucleotide sequence into the genome of a cell. In such embodiments, two homologous recombination procedures are performed as described herein to introduce the desired nucleotide sequence into both copies of the chromosomal target sequence. Alternatively, a genetically modified organism in which one copy of the chromosomal target sequence has been modified as desired may be generated using the methods described herein. Subsequently, cells may be obtained from the genetically modified organism and subjected to a second homologous recombination procedure as described herein. The cells from the second homologous recombination procedure may then be used to generate an organism in which both chromosomal copies of the target sequence have been modified as desired.

In some embodiments, the insertion sequence or a portion thereof may be located between two sites, such as loxP sites, which allow the insertion sequence or a portion thereof to be deleted from the genome of the cell at a desired time. In embodiments in which the insertion sequence or a portion thereof is located between loxP sites, the insertion sequence or portion thereof may be removed from the genome of the cell by providing the Cre protein. Cre may be provided in the cells in which a homologous recombination event has occurred by introducing Cre into the cells through lipofection (Baubonis et al., 1993, Nucleic Acids Res. 21:2025-9), or by transfecting the cells with a vector comprising an inducible promoter operably linked to a nucleic acid encoding Cre (Gu et al., 1994, Science 265:103-106).

In some embodiments, the recombination vector comprises a nucleotide sequence which encodes a detectable or selectable marker which facilitates the identification or selection of cells in which the desired homologous recombination event has occurred. For example, the detectable marker may be a cell surface protein which is recognized by an antibody such that cells expressing the cell surface marker may be isolated using FACS. Alternatively, the recombination vector may comprise a selectable marker which provides resistance to a drug.

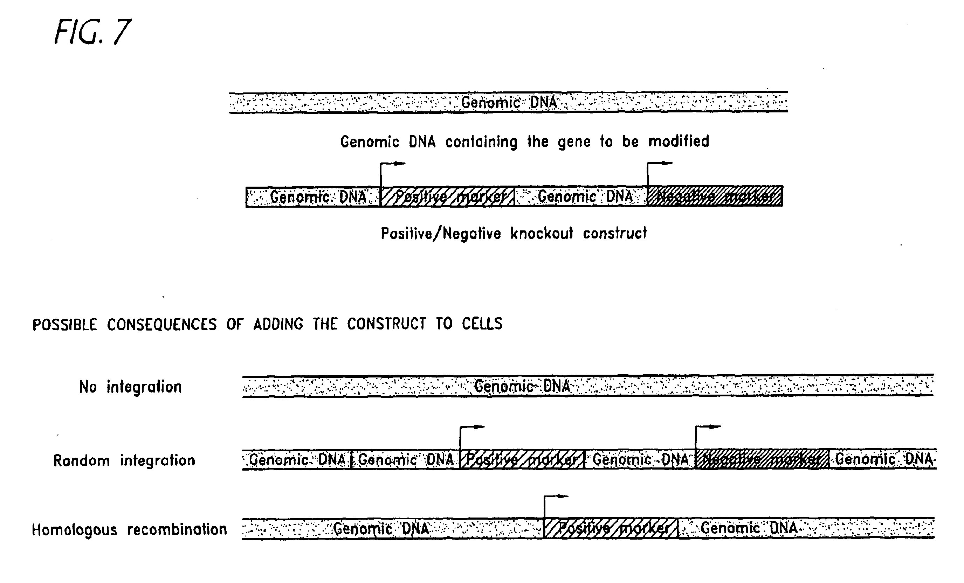

The recombination vector may be introduced into the cell concurrently with the ZFE, prior to the ZFE, or after the ZFE. Cleavage of the chromosomal DNA by the ZFE enhances the frequency of homologous recombination by the recombination vector. Cells in which the desired recombination event has occurred are identified and, if desired, the chromosomal structure of the cells may verified using techniques such as PCR or Southern blotting. Further discussion of recombination vectors and methods for their use is provided in Example 6, and several exemplary constructs are provided in Figures 7-9.

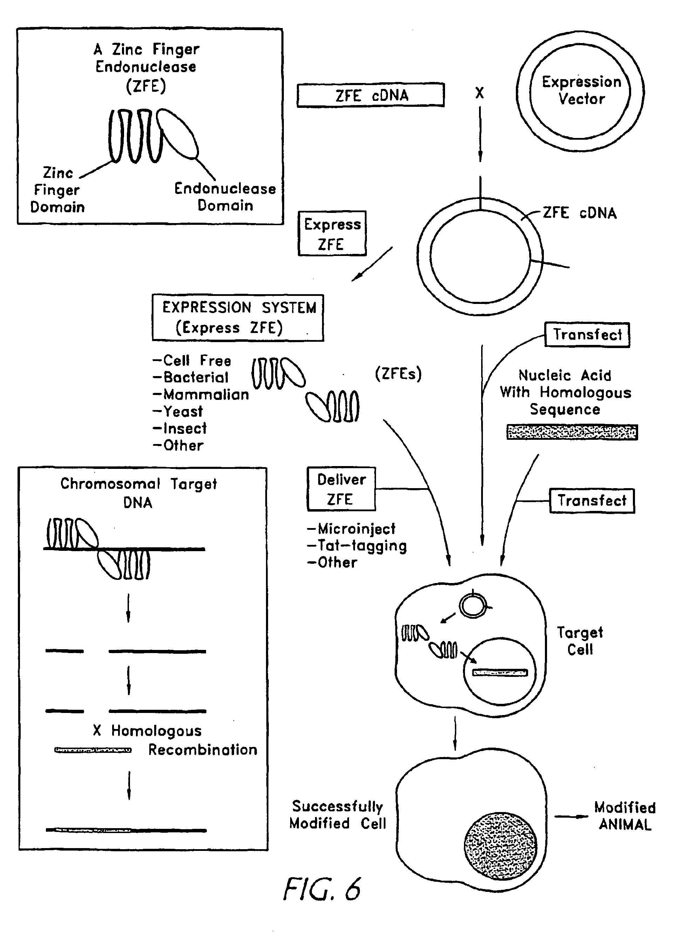

Figure 6 illustrates a method for comparison.

The following examples are intended to illustrate some embodiments of the present invention. It will be appreciated that the following examples are exemplary only and that the scope of the present invention is defined by the appended claims. In particular it will be appreciated that any methodologies familiar to those skilled in the art may be substituted for those specifically enumerated in the examples below. Further, it will be appreciated that although certain organisms or cells are used in the following examples, other organisms or cells which are consistent with the intent of the present invention may be submitted.

### EXAMPLES

### Example 1 (for comparison)

### DESIGN OF A ZINC FINGER ENDONUCLEASE

A ZFE is designed with an endonuclease domain that cuts DNA and a zinc finger domain which recognizes the specific DNA sequence "GTGGCAGCC" (SEQ ID NO: 12). The zinc finger domains encoded by the sequence illustrated in Figure 4 are fused to the Fok I endonuclease.

A standard PCR protocol is performed using the primers illustrated in Figure 5 in order to make and amplify the zinc finger domain encoded by the sequence in Figure 4. The Fok I sequence illustrated in Figure 2 is amplified using standard PCR methods. The amplified zinc finger domain sequence is joined to the amplified Fok I construct thereby forming a chimeric DNA sequence.

### Example 2 (inventive)

### DESIGN OF 6-MER ENDONUCLEASE DOMAIN

The zinc finger coding domains of Figure 4 are cut using the restriction sites Age I and Xma I. The two three-finger coding domains are joined to form a six-finger coding domain with the same consensus linker (TGEKP; SEQ ID NO: 13) between all fingers. This six finger framework is linked to the HO endonuclease domain illustrated in Figure 1.

### Example 3 (inventive)

### DESIGN OF A SEQUENCE SPECIFIC ZFE

A target endogenous chromosomal nucleotide sequence at or near which it is desired to enhance the frequency of homologous recombination is identified and scanned to identify a sequence which will be bound by a zinc finger protein comprising 6 zinc finger domains. If "N" is any base pair, then the zinc fingers are selected to bind to the following sequence within the target nucleic acid: 5'- G/A N N G/A N N G/A N N G/A N N G/A N N G/A N N-3' (SEQ ID NO: 11), where N is A, G, C or T.

### Example 4 (for comparison)

### DESIGN OF A SEQUENCE SPECIFIC ZFE:

A target endogenous chromosomal target sequence at or near which it is desired to enhance the frequency of homologous recombination is identified and scanned to identify a nucleotide sequence which will be recognized by a ZFE. Two 3-mer zinc finger domains for use with the Fok I endonuclease are designed by determining a zinc finger protein that will specifically bind to the target DNA in a mirror image orientation, with a space of 6 bp in between the two. If "N" is A, G, C or T, then all of the zinc fingers that bind to any sequence "GNN" and "ANN" are known. The zinc finger domain is selected to bind to the sequence 5'-G/A N N G/A N N G/A N N N N N N N N N N C/T N N C/T N N C/T-3' (SEQ ID NO: 10).

### Example 5

### EXPRESSION OF THE ZFE

The construct of Example 1 (for comparison) or 2 (inventive) is introduced into the pMal bacterial expression vector (New England Biolabs) and expressed. The ZFE protein is expressed under the control of the malE promoter in bacteria tagged with a maltose binding protein. The ZFE protein is purified by maltose chromatography and quantified.

### Example 6

### GENERATION OF A COW CELL IN WHICH BOTH CHROMOSOMAL COPIES OF A TARGET GENE ARE DISRUPTED

ZFE protein from Example 5 is microinjected into a primary cow cell. A range of concentrations of ZFE protein is injected. In some embodiments, this range is approximately 5-10 mg of protein per ml of buffer injected, but any concentration of ZFE which is sufficient to enhance the frequency of homologous recombination may be used. Also, a recombination vector containing the target gene or a portion thereof in which the coding sequence has been disrupted is introduced into the cow cell. In some embodiments, the vector is introduced at a concentration of about 100ng/µl, but any concentration which is sufficient to permit homologous recombination may be used. Both the DNA and the ZFE protein are resuspended in a buffer, such as 10mM HEPES buffer (pH 7.0) which contains 30mM KCl. The homologous recombination construct containing the disrupted coding sequence is either introduced into the cell by microinjection with the ZFE protein or using techniques such as lipofection or calcium phosphate transfection.

Homologous recombination is the exchange of homologous stretches of DNA. In order to alter the genome by homologous recombination, DNA constructs containing areas of homology to genomic DNA are added to a cell. One challenge associated with homologous recombination is that it normally occurs rarely. A second problem is that there is a relatively high rate of random integration into the genome. (Capecchi, "Altering the genome by homologous recombination," Science 244 (4910):1288-1292 (1989)). The inclusion of ZFEs increases the rate of homologous recombination while the rate of random integration is unaffected.

A number of different DNA construct designs can be used to distinguish homologous recombination from random integration, thereby facilitating the identification of cells in which the desired homologous recombination has occurred. Several exemplary DNA constructs used for homologous recombination are provided below. The first three ("Positive/Negative selection constructs," "Gene Trapping constructs," and "Overlapping constructs") all provide methods that allow homologous recombination to be efficiently distinguished from random integration.

### Positive/Negative Knockout Construct

One type of construct used is a Positive/Negative Knockout Construct. In this construct a "positive" marker is one that indicates that the DNA construct has integrated somewhere in the genome. A "negative" marker is one that indicates that the DNA construct has integrated at random in the genome, (Hanson et al., "Analysis of biological selections for high-efficiency gene targeting," Mol.Cell Biol. 15 (1):45-51 (1995)).

The "positive" marker is a gene under the control of a constitutively active promoter, for example the promoters of Cyto MegaloVirus (CMV) or the promoter of Simian Virus 40 (SV40). The gene controlled in this way may be an auto-fluorescent protein such as, for example, Enhance Green Fluorescent Protein (EGFP) or DsRed2 (both from Clontech), a gene that encodes resistance to a certain antibiotic (neomycin resistance or hygromycin resistance), a gene encoding a cell surface antigen that can be detected using commercially available antibody, for example CD4 or CD8 (antibodies raised against these proteins come from Rockland, Pharmingen or Jackson), and the like.

The "negative" marker is also a gene under the control of a constitutively active promoter like that of CMV or SV40. The gene controlled in this way may also be an auto-fluorescent protein such as EGFP or DsRed2 (Clontech), a gene that encodes resistance to a certain antibiotic (neomycin resistance or hygromycin resistance) a gene encoding a cell surface antigen that can be detected by antibodies, and the like. However, the "negative" marker may also be a gene whose product either causes the cell to die by apoptosis, for example, or changes the morphology of the cell in such a way that it is readily detectable by microscopy, for example E-cadherin in early blastocysts.

The "positive" marker is flanked by regions of DNA homologous to genomic DNA. The region lying 5' to the "positive" marker can be about I kB in length, to allow PCR analysis using the primers specific for the "positive" marker and a region of the genome that lies outside of the recombination construct, but may have any length which permits homologous recombination to occur. If the PCR reaction using these primers produces a DNA product of expected size, this is further evidence that a homologous recombination event has occurred. The region to the 3' of the positive marker can also have any length which permits homologous recombination to occur. Preferably, the 3' region is as long as possible, but short enough to clone in a bacterial plasmid. For example, the upper range for such a stretch of DNA can be about 10 kB in some embodiments. This 3' flanking sequence can be at least 3 kB. To the 3' end of this stretch of genomic DNA the "negative" marker is attached.

Once this DNA has been introduced into the cell, the cell will fall into one of three phenotypes: (1) No expression of either the "positive" or "negative" marker, for example, where there has been no detectable integration of the DNA construct. (2) Expression of the "positive" and "negative" markers. There may have been a random integration of this construct somewhere within the genome. (3) Expression of the "positive" marker but not the "negative" marker. Homologous recombination may have occurred between the genomic DNA flanking the "positive" marker in the construct and endogenous DNA. In this way the "negative" marker has been lost. These are the desired cells. These three possibilities are shown schematically in Figure 7.

### Gene Trapping Construct

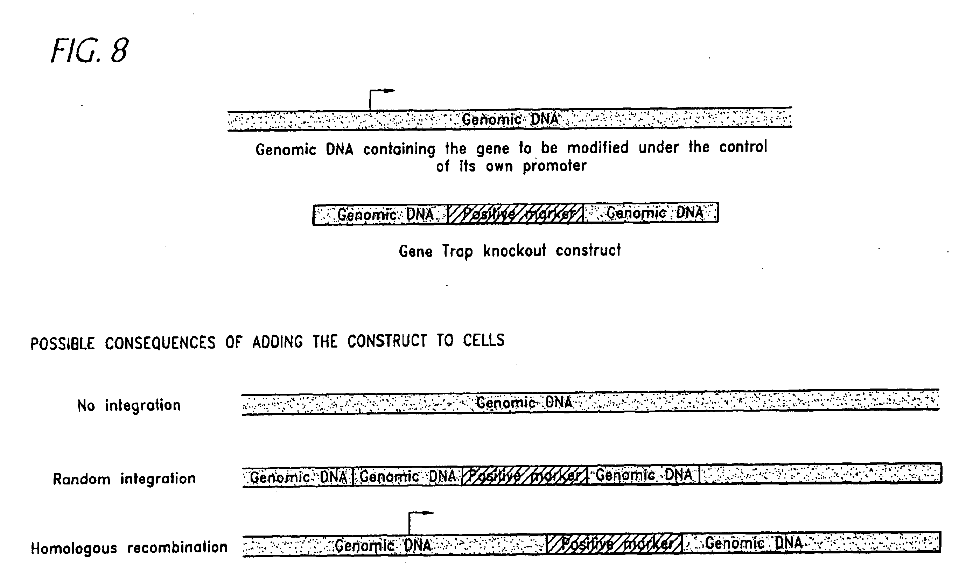

Another type of construct used is called a "Gene Trapping construct." These constructs contain a promoter-less "positive" marker gene. This gene may be, for example, any of the genes mentioned above for a positive/negative construct. This marker gene is also flanked by pieces of DNA that are homologous to genomic DNA. In this case however, 5' flanking DNA must put the marker gene under the control of the promoter of the gene to be modified if homologous recombination happens as desired (Sedivy et al., "Positive genetic selection for gene disruption in mammalian cells by homologous recombination," Proc.Natl.Acad.Sci.U.S.A 86 (1):227-231 (1989)). Preferably, this 5' flanking DNA does not drive expression of the "positive" marker gene by itself. One possible way of doing this is to make a construct where the marker is in frame with the first coding exon of the target gene, but does not include the actual promoter sequences of the gene to be modified. It should be noted that, in preferred embodiments, this technique works if the gene to be modified is expressed at a detectable level in the cell type in which homologous recombination is being attempted. The higher the expression of the endogenous gene the more likely this technique is to work. The region 5' to the marker can also have any length that permits homologous recombination to occur. Preferably, the 5' region can be about 1 kB long, to facilitate PCR using primers in the marker and endogenous DNA, in the same way as described above. Similarly, preferably the 3' flanking region can contain as long a region of homology as possible. An example of an enhancer trapping knockout construct is shown in Figure 8.

These enhancer trapping based knockout constructs may also contain a 3' flanking "negative" marker. In this case the DNA construct can be selected for on the basis of three criteria, for example. Expression of the "positive" marker under the control of the endogenous promoter, absence of the "negative" marker, and a positive result of the PCR reaction using the primer pair described above.

### Over-lapping Knockout Construct

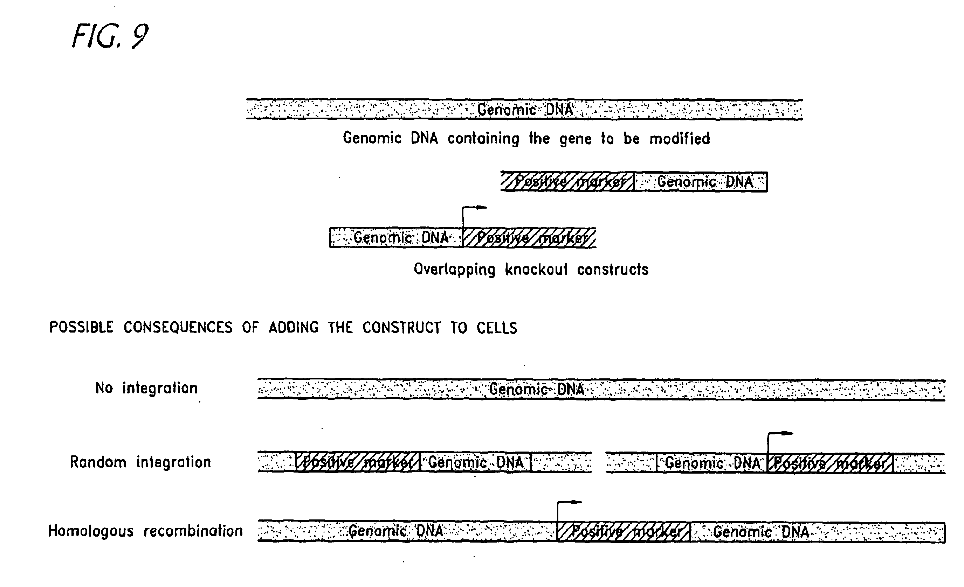

A further type of construct is called an "Over-lapping knockout construct." This technique uses two DNA constructs (Jallepalli et al., "Securin is required for chromosomal stability in human cells," Cell 105 (4):445-457 (2001)). Each construct contains an overlapping portion of a "positive" marker, but not enough of the marker gene to make a functional reporter protein on its own. The marker is composed of both a constitutively active promoter, for example CMV or SV40 and the coding region for a "positive" marker gene, such as for example, any of those described above. In addition to the marker gene, each of the constructs contains a segment of DNA that flanks the desired integration site. The region of the gene replaced by the "positive" marker is the same size as that marker. If both of these constructs integrate into the genome in such a way as to complete the coding region for the "positive" marker, then that marker is expressed. The chances that both constructs will integrate at random in such an orientation are negligible. Generally, if both constructs integrate by homologous recombination, is it likely that a functional coding region for the "positive" marker will be recreated, and its expression detectable. An example of an overlapping knockout construct is shown in Figure 9.

### Stopper Construct

Another DNA construct, called a "stopper construct," enhances the rate of homologous recombination, but does not contain an intrinsic means of distinguishing homologous recombination from random integration. Unlike the other constructs this one contains no marker genes either "positive" or "negative." The construct is a stretch of DNA homologous to at least part of the coding region of a gene whose expression is to be removed. The only difference between this piece of DNA and its genomic homolog is that somewhere in region of this DNA that would normally form part of the coding region of the gene, the following sequence, herein referred to as a "stopper sequence," has been substituted: 5'-ACTAGTTAACTGATCA-3' (SEQ ID NO: 14). This DNA sequence is 16 bp long, and its introduction adds a stop codon in all three reading frames as well as a recognition site for SpeI and BclI. BclI is methylated by Dam and Dcm methylase activity in bacteria.

Integration by homologous recombination is detectable in two ways. The first method is the most direct, but it requires that the product of the gene being modified is expressed on the surface of the cell, and that there is an antibody that exists that recognizes this protein. If both of these conditions are met, then the introduction of the stop codons truncates the translation of the protein. The truncation shortens the protein so much that it is no longer functional in the cell or detectable by antibodies (either by FACS of Immuno-histochemistry). The second indirect way of checking for integration of the "stopper construct" is PCR based. Primers are designed so that one lies outside of the knockout construct, and the other lies within the construct past the position of the "stopper sequence." PCR will produce a product whether there has been integration or not. A SpeI restriction digest is carried out on the product of this PCR. If homologous recombination has occurred the "stopper construct" will have introduced a novel SpeI site that should be detectable by gel electrophoresis.

Integration of any of the constructs described above by homologous recombination can be verified using a Southern blot. Introduction of the construct will add novel restriction endonuclease sites into the target genomic DNA. If this genomic DNA is digested with appropriate enzymes the DNA flanking the site of recombination is contained in fragments of DNA that are a different size compared to the fragments of genomic DNA which have been digested with the same enzymes but in which homologous recombination has not occurred. Radioactive DNA probes with sequences homologous to these flanking pieces of DNA can be used to detect the change in size of these fragments by Southern blotting using standard methods.

Using either the "Positive/negative", "Gene Trap" or "Over-lapping" strategies described above, the genetically modified cell ends up with an exogenous marker gene integrated into the genome. In any of these strategies the marker gene and any exogenous regulatory sequences may be flanked by LoxP recombination sites and subsequently removed.

Removal occurs because recombination may occur between two LoxP sites which excises the intervening DNA (Sternberg et al., "Bacteriophage P1 site-specific recombination. II. Recombination between loxP and the bacterial chromosome," J.Mol.Biol. 150 (4):487-507 (1981); and Sternberg et al., "Bacteriophage P1 site-specific recombination. I. Recombination between loxP sites," J.Mol.Biol. 150 (4):467-486 (1981)). This recombination is driven by the Cre recombinase (Abremski et al., "Bacteriophage P1 site-specific recombination. Purification and properties of the Cre recombinase protein," J.Bio/.Chem. 259 (3):1509-1514 (1984)). This can be provided in cells in which homologous recombination has occurred by introducing it into cells through lipofection (Baubonis et al., "Genomic targeting with purified Cre recombinase," Nucleic Acids Res. 21 (9):2025-2029 (1993)), or by transfecting the cells with a vector comprising an inducible promoter linked to DNA encoding Cre recombinase (Gu et al., "Deletion of a DNA polymerase beta gene segment in T cells using cell type-specific gene targeting," Science 265 (5168):103-106 (1994)).

It will be appreciated that the recombination vector may include any sequence, which sequence one desires to introduce into the genome using homologous recombination. For example, if one desires to disrupt a gene in the genome of the cell, the genomic sequence homologous to the target chromosomal sequence may comprise a stop codon in the coding sequence of the target gene. Alternatively, as discussed above, the recombination vector may contain a gene which rescues a defect in the endogenous target gene or a gene from another organism which one desires to express. Alternatively, the recombination vector may contain a sequence which introduces a deletion in the target gene.

If both functional copies of a gene have been disrupted, then the "stopper construct described above has worked. It will also be appreciated that the "Positive/Negative", "Gene Trap" and "Overlapping constructs" described above may be used twice if one desires to introduce a genetic modifications at both copies of the endogenous target sequence. The main modification is that the second time these constructs are used to knockout a gene, the "positive" marker in each case should be distinguishable from the "positive" marker used in the constructs to knock out the first copy of the gene.

### Example 7

### GENERATION OF A GENETICALLY MODIFIED ORGANISM

Nuclear transfer using nuclei from cells obtained as described in Example 6 is performed as described by Wilmut et al., Nature 385(6619)810-813 (1997), U.S. Patent Number 6,147,276, U.S. Patent Number 5,945,577 or U.S. Patent Number 6,077,710. Briefly, the nuclei are transferred into enucleated fertilized oocytes. A large number of oocytes are generated in this manner. Approximately ten animals are fertilized with the oocytes, with at least six fertilized embryos being implanted into each animal and allowed to progress through birth.

Animals and/or plants comprising cells, organs or tissues containing the desired genetic modifications may also be generated using other methods familiar to those skilled in the art. For example, as discussed above, stem cell-based technologies may be employed.

### Example 8

### GENERATION OF A GENETICALLY MODIFIED PLANT

Homologous recombination methods are also useful to introduce genetic changes into plant cells, which can then be used, for example, for research or for regenerating whole plants for agricultural purposes. To perform homologous recombination in a plant cell, a suitable endogenous chromosomal target sequence is first chosen, and a ZFE which recognizes a specific nucleotide sequence within that target sequence is designed. Additionally, a nucleic acid fragment that is homologous to at least a portion of the endogenous chromosomal target sequence is prepared. A suitable vector containing the ZFE sequence may be constructed and introduced into the plant cell by various means, along with the prepared homologous nucleic acid fragment to be inserted. It should be noted that in some embodiments the ZFE can be expressed outside of the plant cell, and then the protein can be introduced into the plant cell. Once produced inside the plant cell (or introduced into the plant cell), the ZFE binds to the specified nucleic acid site on the target sequence, and subsequently performs a double stranded cut in the target sequence. Upon the introduction of the prepared homologous nucleic acid fragment, homologous recombination occurs.

One of skill in the art can select an appropriate vector for introducing the ZFE-encoding nucleic acid sequence in a relatively intact state. Thus, any vector which produces a cell or a plant carrying the introduced DNA sequence is sufficient. Even a naked piece of DNA encoding the ZFE may be used to express the ZFE in the cell or Plant.

In one method, the ZFE gene is cloned into a suitable expression vector capable of expressing the gene in plant cells. The expression vector is typically amplified in a bacterial host cell culture, and purified by conventional means known to one of skill in the art. A variety of host-expression vector systems may be utilized to express the ZFE coding sequence in plant cells. Examples include but are not limited to plant cell systems infected with recombinant virus expression vectors (e.g., cauliflower mosaic virus, CaMV; tobacco mosaic virus, TMV) or transformed with recombinant plasmid expression vectors containing the ZFE coding sequence.

To be effective once introduced into plant cells, the ZFE encoding nucleic acid sequence is preferably associated with a promoter which is effective in driving transcription of the ZFE gene in plant cells. Any of a number of promoters may be suitable, such as constitutive promoters, inducible promoters, and regulatable promoters. For plant expression vectors, suitable viral promoters include but are not limited to the 35S RNA and 19S RNA promoters of CaMV (Brisson, et al., Nature, 310:511, 1984; Odell, et al., Nature, 313:810, 1985); the full-length transcript promoter from Figwort Mosaic Virus (FMV) (Gowda, et al., J. Cell Biochem., 13D: 301, 1989) and the coat protein promoter to TMV (Takamatsu, et al., EMBO J. 6:307, 1987). Alternatively, plant promoters such as the light-inducible promoter from the small subunit of ribulose bis-phosphate carboxylase (ssRUBISCO) (Coruzzi, et al., EMBO J., 3:1671, 1984; Broglie, et al., Science, 224:838, 1984); mannopine synthase promoter (Velten, et al., EMBO J., 3:2723, 1984) nopaline synthase (NOS) and octopine synthase (OCS) promoters (carried on tumor-inducing plasmids of *Agrobacterium tumefaciens*) or heat shock promoters, e.g., soybean hsp 17.5-E or hsp17.3-B (Gurley, et al., Mol. Cell. Biol., 6:559, 1986; Severin, et al., Plant Mol. Biol., 15:827, 1990) may be used. Additionally, a polyadenylation sequence or transcription control sequence recognized in plant cells may be employed.

Optionally, a selectable marker may be associated with the ZFE nucleic acid sequence to be introduced to the plant cell. As used in this example, the term "marker" refers to a gene encoding a trait or a phenotype which permits the selection of, or the screening for, a plant or plant cell containing the marker. The marker gene may be an antibiotic resistance gene whereby the appropriate antibiotic can be used to select for cells that have taken up the vector containing the ZFE gene. Examples of suitable selectable markers include adenosine deaminase, dihydrofolate reductase, hygromycin-B-phospho-transferase, thymidine kinase, xanthine-guanine phosphoribosyltransferase and amino-glycoside 3'-O-phospho-transferase II (kanamycin, neomycin and G418 resistance). Other suitable markers are known to those of skill in the art.

Genetically modified plants of the present invention may be produced by contacting a plant cell with the above-described expression vector comprising a nucleic acid encoding the ZFE protein. One method for introducing the ZFE expression vector to plant cells utilizes electroporation techniques. In this technique, plant protoplasts are prepared following conventional methods (i.e., Shillito and Saul, (1988) Protoplast isolation and transformation in Plant Molecular Biology - A Practical Approach (C.H. Shaw, Ed.; IRL Press) 161-186). The protoplasts are then electroporated in the presence of the ZFE-encoding expression vector. Electrical impulses of high field strength reversibly permeabilize membranes allowing the introduction of nucleic acids.

Alternatively, the ZFE-encoding expression vector can also be by means of high velocity microparticle bombardment techniques to transfer small particles with the nucleic acid to be introduced contained either within the matrix of such particles, or on the surface thereof to the inside of the plant cell (Klein, et al., Nature 327:70, 1987). Microparticle bombardment methods are also described in Sanford, et al. (Techniques 3:3, 1991) and Klein, et al. (Bio/Techniques 10:286, 1992).

The homologous nucleic acid fragment to be inserted may also be introduced into the plant cell using microparticle bombardment or electroporation techniques as described herein. The nucleic acid fragment to be inserted into the genome may be transferred to the cell at the same time and method as the expression vector (or the expressed ZFE), or it may be transferred to the cell prior or subsequent to the transfer of the expression vector (or the expressed ZFE). The nucleic acid to be inserted into the genome may be included in any of the recombination vectors described above. Likewise, the nucleic acid to be inserted into the genome may have any of the characteristics or features described above.

During and after the homologous recombination process described above, the electroporated plant protoplasts typically reform the cell wall, divide and form a plant callus. The callus may be regenerated into plantlets and whole, mature plants, if desired. Alternatively, the protoplasts may be cultured as suspension of single intact cells in a solution. Methods of testing for the success of the homologous recombination, as well as methods for selecting for cells transformed by the above-described homologous transformation procedure, may then be performed.

## Claims

1. An *in vitro* method of altering the genome of an isolated cell by homologous recombination, the cell comprising an endogenous chromosomal target DNA sequence which is present only once in the genome, the method, comprising:
contacting said cell with a zinc finger endonuclease or with a polynucleotide encoding a zinc finger endonuclease, wherein the zinc finger endonuclease comprises an endonuclease domain and a zinc finger domain comprising five or more zinc fingers that binds to a specific nucleotide sequence within said endogenous chromosomal target DNA in said cell, under conditions such that said zinc finger endonuclease cuts both strands of a nucleotide sequence within said endogenous chromosomal target DNA sequence in said cell; and
introducing into said cell a nucleic acid comprising a sequence homologous to at least a portion of said endogenous chromosomal target DNA such that homologous recombination occurs between said endogenous chromosomal target DNA sequence and said nucleic acid, wherein the endonuclease domain is HO endonuclease.

2. The *in vitro* method of claim 1, wherein one or more of zinc finger endonucleases further comprises a protein tag to purify the resultant protein.

3. The *in vitro* method of claim 2, wherein said protein tag is selected from the group consisting of HA tag, FLAG-tag, GST-tag, c-myc, and His-tag.

4. The *in vitro* method of claim 1, wherein said contacting comprises transfecting said cell with a vector comprising a sequence encoding said one or more zinc finger endonucleases, such that said one or more zinc finger endonucleases are expressed in the cell.

5. The *in vitro* method of claim 1, wherein said contacting comprises introducing a zinc finger endonuclease protein into said cell.

6. The *in vitro* method of claim 1, wherein each of said plurality of zinc fingers binds to the sequence G/ANN.

7. The *in vitro* method of claim 1, wherein said cell is from an organism selected from the group consisting of plant, a mammal, a marsupial, an avian and teleost fish.

8. The *in vitro* method of claim 7, wherein said mammal is selected from the group consisting of a non-human primate, a sheep, a goat, a cow, a rat and a pig.

9. The *in vitro* method of claim 7, wherein said mammal is a mouse.

10. The *in vitro* method of claim 1, wherein said cell is a primary cell or a non-human stem cell.

11. A method of generating a genetically modified animal in which a desired nucleic acid has been introduced, comprising:
generating an animal from a cell whose genome has been altered by the method of any of claims 1-10, wherein animal does not comprise human, wherein said animal is selected from the group consisting of a mammal, a marsupial, an avian, and teleost fish, wherein said zinc finger domain comprises at least 5 zinc fingers.

12. The method of claim 11, wherein said mammal is selected from the group consisting of a non-human primate, a sheep, a goat, a cow, a rat and a pig.

13. The method of claim 11, wherein said mammal is a mouse.

14. The method of claim 11, wherein said nucleic acid introduced into said cell comprises a nucleotide sequence selected from the group consisting of a nucleotide sequence which disrupts a gene after homologous recombination, a nucleotide sequence which replaces a gene after homologous recombination, a nucleotide sequence which introduces a point mutation into a gene after homologous recombination, and a nucleotide sequence which introduces a regulatory site after homologous recombination.

15. The method of claim 14, wherein said regulatory site comprises a LoxP site.

16. A method of generating a genetically modified plant in which a desired nucleic acid has been introduced, comprising:
generating a plant from a cell whose genome has been altered by the method of any of claims 1-6.

## Patentansprüche

1. *In vitro* Verfahren zur Veränderung des Genoms einer isolierten Zelle durch homologe Rekombination, wobei die Zelle eine endogene chromosomale Ziel-DNA-Sequenz umfasst, die nur einmal im Genom vorliegt, wobei das Verfahren umfasst:
Inkontaktbringen der Zelle mit einer Zinkfinger-Endonuklease oder mit einem Polynukleotid, welches für eine Zinkfinger-Endonuklease kodiert, wobei die ZinkfingerEndonuklease eine Endonukleasedomäne und eine Zinkfingerdomäne,
umfassend fünf oder mehr Zinkfinger, umfasst, die an eine spezifische Nukleotidsequenz innerhalb der endogenen chromosomalen Ziel-DNA in der Zelle bindet, unter solchen Bedingungen, dass die Zinkfinger-Endonuklease beide Stränge der Nukleotidsequenz innerhalb der endogenen chromosomalen Ziel-DNA-Sequenz in der Zelle schneidet; und Einführen einer Nukleinsäure, welche eine Sequenz umfasst, die mindestens zu einem Teilbereich homolog zu der endogenen chromosomalen Ziel-DNA ist, in die Zelle, so dass homologe Rekombination zwischen der endogenen chromosomalen Ziel-DNA-Sequenz und der Nukleinsäure stattfindet, wobei die Endonukleasedomäne eine HO-Endonuklease ist.

2. *In vitro* Verfahren nach Anspruch 1, wobei eine oder mehrere der Zinkfinger-Endonukleasen ferner einen Proteintag zur Aufreinigung des resultierenden Proteins umfassen.

3. *In vitro* Verfahren nach Anspruch 2, wobei der Proteintag aus der Gruppe bestehend aus HA-tag, FLAG-tag, GST-tag, c-myc, und His-tag ausgewählt ist.

4. *In vitro* Verfahren nach Anspruch 1, wobei das Inkontaktbringen die Transfizierung der Zelle mit einem Vektor, umfassend eine Sequenz, die für die eine oder mehrere Zinkfinger-Endonukleasen kodiert, umfasst, so dass die eine oder mehreren Zinkfinger-Endonukleasen in der Zelle exprimiert werden.

5. *In vitro* Verfahren nach Anspruch 1, wobei das Inkontaktbringen die Einführung eines Zinkfinger-Endonuklease Proteins in die Zelle umfasst.

6. *In vitro* Verfahren nach Anspruch 1, wobei jeder von der Vielzahl an Zinkfingern an die Sequenz G/ANN bindet.

7. *In vitro* Verfahren nach Anspruch 1, wobei die Zelle von einem Orgnismus ist, der aus der Gruppe bestehend aus einer Pflanze, einem Säuger, einem Beuteltier, einem Vogel und einem Knochenfisch, ausgewählt ist.

8. *In vitro* Verfahren nach Anspruch 7, wobei der Säuger aus der Gruppe bestehend aus einem nicht-humanen Primaten, einem Schaf, einer Ziege, einer Kuh, einer Ratte und einem Schwein ausgewählt ist.

9. *In* vitro-Verfahren nach Anspruch 7, wobei der Säuger eine Maus ist.

10. *In vitro* Verfahren nach Anspruch 1, wobei die Zelle eine Primärzelle oder eine nichthumane Stammzelle ist.

11. Verfahren zur Herstellung eines genetisch modifizierten Tieres, in welches eine gewünschte Nukleinsäure eingeführt worden ist, umfassend:
Herstellung eines Tieres aus einer Zelle, deren Genom durch das Verfahren nach einem der Ansprüche 1 bis 10 verändert worden ist, wobei das Tier keinen Menschen umfasst, wobei das Tier aus der Gruppe bestehend aus einem Säuger, einem Beuteltier, einem Vogel, und einem Knochenfisch ausgewählt ist, wobei die Zinkfingerdomäne mindestens 5 Zinkfinger umfasst.

12. Verfahren nach Anspruch 11, wobei der Säuger aus der Gruppe bestehend aus einem nicht-humanen Primaten, einem Schaf, einer Ziege, einer Kuh, einer Ratte und einem Schwein ausgewählt ist.

13. Verfahren nach Anspruch 11, wobei der Säuger eine Maus ist.

14. Verfahren nach Anspruch 11, wobei die in die Zelle eingeführte Nukleinsäure eine Nukleotidsequenz umfasst, die aus der Gruppe bestehend aus einer Nukleotidsequenz, welche ein Gen nach homologer Rekombination unterbricht, einer Nukleotidsequenz, welche ein Gen nach homologer Rekombination ersetzt, einer Nukleotidsequenz, welche eine Punktmutation in ein Gen nach homologer Rekombination einführt, und
einer Nukleotidsequenz, welche eine Regulationsstelle nach homologer Rekombination einführt, ausgewählt ist.

15. Verfahren nach Anspruch 14, wobei die Regulationsstelle eine LoxP Stelle umfasst.

16. Verfahren zur Herstellung einer genetisch modifizierten Pflanze, in welche eine gewünschte Nukleinsäure eingeführt worden ist, umfassend:
Herstellung einer Pflanze aus einer Zelle, deren Genom durch das Verfahren nach einem der Ansprüche 1 bis 6 verändert worden ist.

## Revendications

1. Procédé *in vitro* de modification du génome d'une cellule isolée par recombinaison homologue, la cellule comprenant une séquence d'ADN chromosomique endogène cible qui n'est présente qu'une seule fois dans le génome, le procédé comprenant:
la mise en contact de ladite cellule avec une endonucléase à doigts de zinc ou avec un polynucléotide codant pour une endonucléase à doigts de zinc, l'endonucléase à doigts de zinc comprenant un domaine d'endonucléase et un domaine à doigts de zinc comprenant au moins 5 doigts de zinc qui se lie à une séquence de nucléotides spécifique dans ledit ADN chromosomique endogène cible dans ladite cellule, dans des conditions telles que ladite endonucléase à doigts de zinc coupe les deux brins d'une séquence de nucléotides dans ladite séquence d'ADN chromosomique endogène cible dans ladite cellule; et
l'introduction dans ladite cellule d'un acide nucléique comprenant une séquence homologue à au moins une portion dudit ADN chromosomique endogène cible de façon qu'il se produise une recombinaison homologue entre ladite séquence d'ADN chromosomique endogène cible et ledit acide nucléique,
le domaine d'endonucléase étant l'endonucléase HO.

2. Procédé *in vitro* selon la revendication 1, dans lequel une ou plusieurs des endonucléases à doigts de zinc comprend en outre une protéine tag pour purifier la protéine obtenue.

3. Procédé *in vitro* selon la revendication 2, dans lequel ladite protéine tag est choisie dans le groupe constitué par HA-tag, FLAG-tag, GST-tag, c-myc, et His-tag.

4. Procédé *in vitro* selon la revendication 1, dans lequel ladite mise en contact comprend la transfection de ladite cellule avec un vecteur comprenant une séquence codant pour lesdites une ou plusieurs endonucléases à doigts de zinc, de façon que lesdites une ou plusieurs endonucléases à doigts de zinc soient exprimées dans la cellule.

5. Procédé *in vitro* selon la revendication 1, dans lequel ladite mise en contact comprend l'introduction d'une protéine endonucléase à doigts de zinc dans ladite cellule.

6. Procédé *in vitro* selon la revendication 1, dans lequel chacun desdits plusieurs doigts de zinc se lie à la séquence G/ANN.

7. Procédé *in vitro* selon la revendication 1, dans lequel ladite cellule provient d'un organisme choisi dans le groupe constitué par une plante, un mammifère, un marsupial, un oiseau et un poisson téléostéen.

8. Procédé *in vitro* selon la revendication 7, dans lequel ledit mammifère est choisi dans le groupe constitué par un primate non humain, un mouton, une chèvre, une vache, un rat et un porc.

9. Procédé *in vitro* selon la revendication 7, dans lequel ledit mammifère est une souris.

10. Procédé *in vitro* selon la revendication 1, dans lequel ladite cellule est une cellule primaire ou une cellule souche non humaine.

11. Procédé de génération d'un animal génétiquement modifié dans lequel a été introduit un acide nucléique désiré, comprenant:
la génération d'un animal à partir d'une cellule dont le génome a été modifié par le procédé selon l'une quelconque des revendications 1-10, ledit animal ne comprenant pas l'être humain, ledit animal étant choisi dans le groupe constitué par un mammifère, un marsupial, un oiseau et un poisson téléostéen, ledit domaine à doigts de zinc comprenant au moins 5 doigts de zinc.

12. Procédé selon la revendication 11, dans lequel ledit animal est choisi dans le groupe constitué par un primate non humain, un mouton, une chèvre, une vache, un rat et un porc.

13. Procédé selon la revendication 11, dans lequel ledit mammifère est une souris.

14. Procédé selon la revendication 11, dans lequel ledit acide nucléique introduit dans ladite cellule comprend une séquence de nucléotides choisie dans le groupe constitué par une séquence de nucléotides qui perturbe un gène après recombinaison homologue, une séquence de nucléotides qui remplace un gène après recombinaison homologue, une séquence de nucléotides qui introduit une mutation ponctuelle dans un gène après recombinaison homologue, et une séquence de nucléotides qui introduit un site régulateur après recombinaison homologue.

15. Procédé selon la revendication 14, dans lequel ledit site régulateur comprend un site LoxP.

16. Procédé de génération d'un végétal génétiquement modifié dans lequel a été introduit un acide nucléique désiré, comprenant:
la génération d'un végétal à partir d'une cellule dont le génome a été modifié par le procédé selon l'une quelconque des revendications 1-6.
